# EUROPEAN PATENT SPECIFICATION

(11) **EP 0 440 625 B1**
(45) Date of publication and mention of the grant of the patent: **25.08.1993**
(21) Application number: 89904849.0
(22) Date of filing: 14.04.1989
(51) Int. Cl.: A61K 37/54

(54) **HEPARIN-CONTAINING FORMULATIONS**
HEPARIN ENTHALTENDE FORMULIERUNGEN
FORMULATIONS CONTENANT DE L'HEPARINE

(30) Priority: 14.04.1988 GB 8808810
(43) Date of publication of application: 14.08.1991
(73) Proprietor: MERCK PATENT GmbH, D-64271 Darmstadt (DE)
(72) Inventor: SAWYER, Roy, Dyfed SA19 6TR (GB); POWELL-JONES, Christopher, Llandeilo Dyfed SA19 6UL (GB)
(74) Representative: Austin, Hedley William
(86) International application number: GB8900391
(87) International publication number: WO8909615

(56) References cited:
- EP-A- 0 193 330

## Description

The present invention is concerned with formulations containing heparin, and the use of hyaluronidases.

Heparin and its derivatives have been extensively used as anticoagulants for many years, both in pharmacologically active formulations and in formulations used in the treatment of apparatus, prostheses and transplants which come into contact with blood. The use of heparin as an anticoagulant is reviewed by, for example, Crawford, G.P.M. and Douglas, A.S. 1977 in Recent Advances in Blood Coagulation Ed. Poller, L. published by Churchill Livingstone.

Enzymes which cleave hyaluronic acid, or salts or derivatives thereof, are known as hyaluronidases. Hyaluronidases have previously been proposed for use in many formulations containing pharmacologically active ingredients, because of their ability to modify the permeability of tissue in vivo and therefore to act as "spreading" agents, resulting in enhanced delivery or penetration of the pharmacologically active material.

Hyaluronidases have also been proposed for use in the reduction of myocardial ischaemia (the latter term meaning the irreversible damage of heart cells, as well as necrosis, which occurs consequent on the reduction of flow of blood through the cardiac muscle). However, if hyaluronidases were to be used for this purpose, they would almost certainly come into contact with heparin, which is used ubiquitously in cardiovascular surgery. In this case, the effectiveness of the therapy would be negated because certain hyaluronidases, in particular mammalian testicular hyaluronidase (abbreviated to MTH hereafter), are inhibited by very low levels of heparin.

Formulations containing both hyaluronidase and heparin have previously been proposed; for example, German Patent Specification 2101393 proposes a topical composition, for the treatment of psoriasis and other similar dermatological complaints (or for the treatment of varicose veins, tumours, local burns, haemorrhoids or the after-effects of phlebitis), which comprises a surface enzyme (such as a hyaluronidase), an anti-coagulant (such as heparin, dicoumarin or hirudin), and also a peripheral blood dilating agent (such as acetylcholine chloride or nicotinamide). Such formulations are described as ointments, creams, jellies, aerosols or foams. The type of hyaluronidase is not specified.

As we have described in our prior European specification 0 193 330, there are two basic types of hyaluronidase, which are those which are relatively non-specific and cleave hyaluronic acid, chondroitin and related polysaccharides, and those which specifically cleave hyaluronic acid only. The former are much more widely distributed in nature, being found in mammalian testes, liver and spleen and in certain micro-organisms, while the latter are derived from certain other micro-organisms (such as streptomyces bacteria) and from leeches such as Hirudo medicinalis or leeches of the sub-family Hirudinariinae. These latter type cleave hyaluronic acid at a specific site and are termed endo-beta-glucuronidases.

Despite the previously proposed use of heparin in combination with a hyaluronidase, it has been demonstrated that certain hyaluronidases (in particular, mammalian testicular hyaluronidase) are inhibited competitively by heparin (see Mathews & Dorfman, Physiological Review, 35, pp 381 to 402).

A pre-requisite for use as a drug delivery vehicle is that the hyaluronidase enzyme should not be neutralised or inhibited by contact with blood or plasma. Surprisingly, we have now found that endo-beta-glucuronidases are unlike other hyaluronidases because they are not inhibited by heparin anticoagulant; thus according to the invention heparin and an endo-beta-glucuronidase can be coadministered to a patient without impairment of activity of the endo-beta-glucuronidase.

According to the present invention, therefore, there is provided a pharmaceutical formulation comprising heparin in combination with an endo-beta-glucuronidase. The formulation according to the invention further comprises a pharmacologically acceptable diluent, carrier or excipient therefor.

The formulation according to the invention may be in various forms. For example, it may be in a topically applicable form such as a cream, gel, ointment or aerosol; in this case it may be applied to, for example, an open wound or the like where it is desired to prevent coagulation of the blood. Alternatively, the formulation according to the invention may be a slow-release formulation such as a suppository or depot, or (preferably) an intravenous formulation. When referring to intravenous formulations, we mean formulations which can be injected as single or repeated unit doses by means of a hypodermic syringe or the like, or formulations which can be administered into the bloodstream continuously for a prolonged period, by means of a drip or the like. Such injectable intravenous unit doses are preferably provided in sealed sterile ampoules, in which the formulations according to the invention are present in liquid, frozen or lyophilised form.

In the latter case, the carrier is typically a buffered aqueous saline medium (typically buffered to a pH in the range 3 to 5.5), in which the aqueous carrier is sterile distilled (or otherwise highly purified) water (e.g. of purity at least 99.9% by weight). When the formulation according to the invention is intended for injection as a single or repeated dose, each unit dose preferably contains 20 thousand to 50 thousand units of the endo-beta-glucuronidase, one unit being defined as one microgram of glucose equivalent liberated per hour from hyaluronic acid at optimum pH. Approximately 20 such units correspond to one International Unit (I.U.) as described in 'International standard for hyaluronidase'; J.H.Humphrey,Bull. WHO; 16, pp.291-294, 1957.

When the formulation according to the invention is intended for administration as an intravenous drip, the concentration of the endo-beta-glucuronidase is substantially less than the concentration in a formulation to be administered in discrete unit doses (e.g. in bolus form). For example, the concentration of the endo-beta-glucuronidase in an intravenously administerable composition according to the invention may be 10 to 1000 I.U. per kilogram body weight administered over a 24 hour period.

The concentration of heparin in the formulation according to the invention is typically 500 to 4000 I.U. over a 24 hour period administered subcutaneously, intravenously or by infusion; the endo-beta-glucuronidase and heparin are preferably present in a ratio of one I.U. of endo-beta-glucuronidase per two to six I.U. heparin.

The endo-beta-glucuronidase used in the formulation according to the invention may be derived from leeches of the sub-family Hirudinariinae, as described in more detail in the above-mentioned European patent specification. Alternatively, the endo-beta-glucuronidase may be derived from other species of leech, such as Hirudo medicinalis, or from other suitable sources. Just as in the abovementioned European patent specification, genetically engineered or synthetic equivalents to the endo-beta-glucuronoidase are intended to be encompassed by the term "derived from" leeches.

Heparin is an anticoagulant by virtue of its action against thrombin and other pro-coagulation factors. On the other hand, anticoagulation may also be achieved by plasminogen activator-mediated lysis of fibrin and/or fibrinogen thereby preventing clot formation; examples of such clot lytic agents are tissue-type plasminogen activator and hementin (a fibrinolytic or fibrinogenolytic agent derived from Haementeria ghilianii, as described in U.S. Patent 4390630).

The formulation according to the invention may contain one or more further pharmacologically active ingredients; in a particularly preferred embodiment, the formulation according to the invention contains in addition one or more clot-lytic agent, such as prourokinase, urokinase, hementin, streptokinase or tissue plasminogen activator (tPA), and/or a derivative thereof. The clot-lytic agent is typically present in an amount of 500 to 3000 I.U. per kilogram of body weight.

The present invention has been described in terms of a pharmaceutical formulation containing both heparin and an endo-beta-glucuronidase; the invention further comprises a method of therapeutic treatment of thrombotic events (such as the treatment of myocardial infarctions) in which the heparin and endo-beta-glucuronidase are administered to a patient either simultaneously or successively, optionally together with a clot-lytic agent as described above. The clot-lytic agent may be administered together with heparin and/or together with the endo-beta-glucuronidase, or as a separate administration.

Seen from another aspect, the present invention comprises an endo-beta-glucuronidase for use in therapy of a heparin-treated patient, optionally together with hementin and/or with tissue plasminogen activator.

The present invention is illustrated with reference to the following Examples:

### Example 1

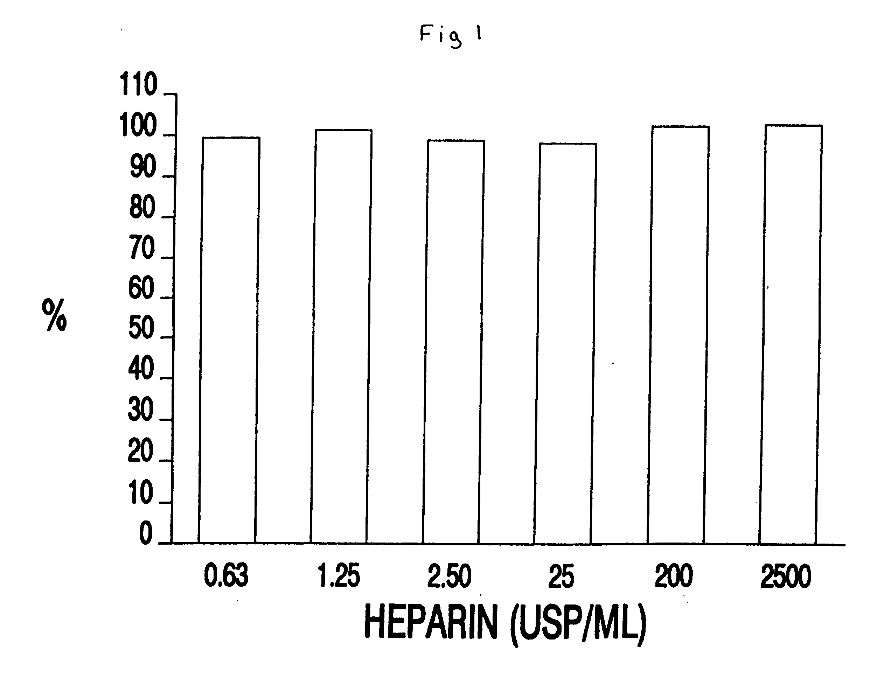

Leech hyaluronidase (68IU/ml was pre-incubated for one hour at 25°C in the presence of concentrations of heparin (0-2500 USP/1ml) in 20mM methanesulphonic acid (MES), 0.1M NaCl, pH 5.0 buffer and then added to hyaluronic acid (5mg/ml) and the incubation continued for one hour at 37°C. The generation of reducing sugars was determined by terminating the reactions by the addition of 1:1 3,5-dinitrosalicylic acid reagent (100ml: 5% w/v dinitrosalicylic acid in 2M NaOH plus 250ml: 60% w/v sodium potassium tartrate in water, made up to 500ml with water) and heating for 5 min in vigorously boiling water. After heating, assay tubes were rapidly cooled to ambient temperature and absorbances at 540nm measured spectrophotometrically. Suitable blanks and no-enzyme controls were included in the assay. The results are expressed as a percentage of the absorbances obtained in control incubations containing no heparin. Concentrations of heparin up to 2500 USP/ml had no significant effect on leech hyaluronidase activity, as indicated in the accompanying Figure 1.

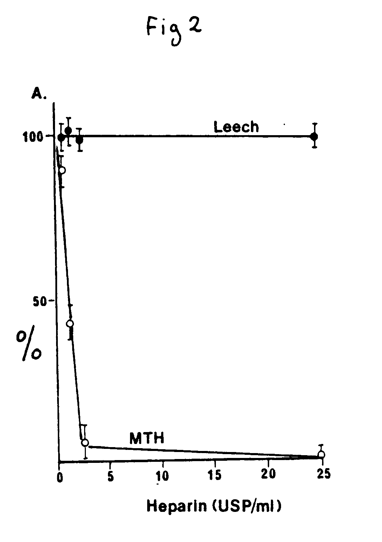

In striking contrast to leech hyaluronidase, mammalian testicular hyaluronidase (MTH) was completely inhibited by levels of heparin above 3 USP/ml (this being a plasma/serum level needed in vivo to induce an uncoagulated state). MTH (75 IU/ml) was pre-incubated for one hour at 25°C with heparin (0 to 250 USP/ml) in 20mM MES, 0.1M NaCl pH 5.0 buffer and then added (1:10 final dilution) to hyaluronate and incubated for a further one hour at 37°C. Activity was determined using the reducing sugar assay (described above) and is expressed as a percentage of controls without heparin. The results obtained with leech hyaluronidase under identical conditions are shown in Figure 2 of the accompanying drawings.

### Example 2

Heparin (0-150 USP/ml) was incubated for one hour at 37°C either with or without leech hyaluronidase (50 IU/ml) and in 20mM MES, 0.1M NaCl, pH5.0 buffer; then whole blood was added to determine the effect on clottability. The results shown in the following Table 1, indicate that the leech hyaluronidase had not affected the ability of heparin to incoagulate blood.

**Table 1**

| Heparin (USP/ml) | Whole Blood Clotting Time (min) | |
|---|---|---|
| | without Leech Enzyme | Leech Enzyme (50IU/ml) |
| 20 | NC | NC |
| 10 | NC | NC |
| 5 | NC | NC |
| 2.5 | 24.0 | 24.5 |
| 1.0 | 14.5. | 14.5 |
| 0 | 4.5 | - |
| NC = no clot formation before 20 minutes at 37°C. | | |

### Example 3

Leech hyaluronidase (100 IU/ml) was mixed 1:1 with Indian ink in 20mM MES, 0.1M NaCl pH5.0 buffer in the presence or absence of heparin (250 to 2500 USP/ml). Rats were injected subcutaneously with 50 microlitres of this mixture and spreading allowed to occur over a period of 1 to 3 hours. The rats were sacrificed and the skin removed for observation of the area of spreading.

The results, summarised in Table 2, show that in the presence of enzyme there was approximately a tenfold increase in the area of ink spreading compared to controls and this was not significantly affected by relatively high doses of heparin.

**Table 2**

| | Heparin (USP/ml) | Area of spreading |
|---|---|---|
| Without enzyme | 0 ) | Range 0.3 to 0.6 cm² |
| | 250 ) | |
| | 2500 ) | |
| With enzyme | 0 ) | Range 2.0 to 4.0 cm² |
| | 250 ) | |
| | 2500 ) | |

### Example 4

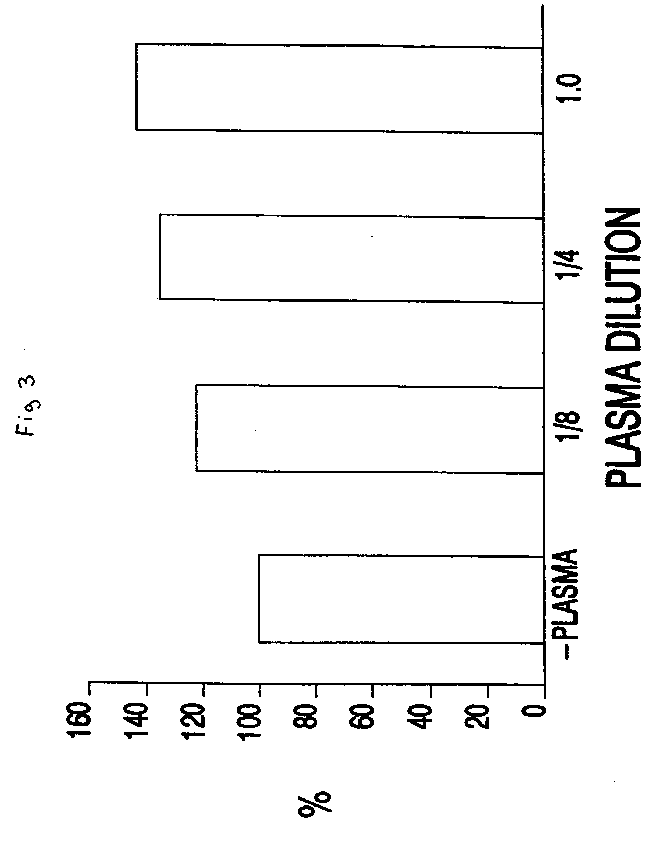

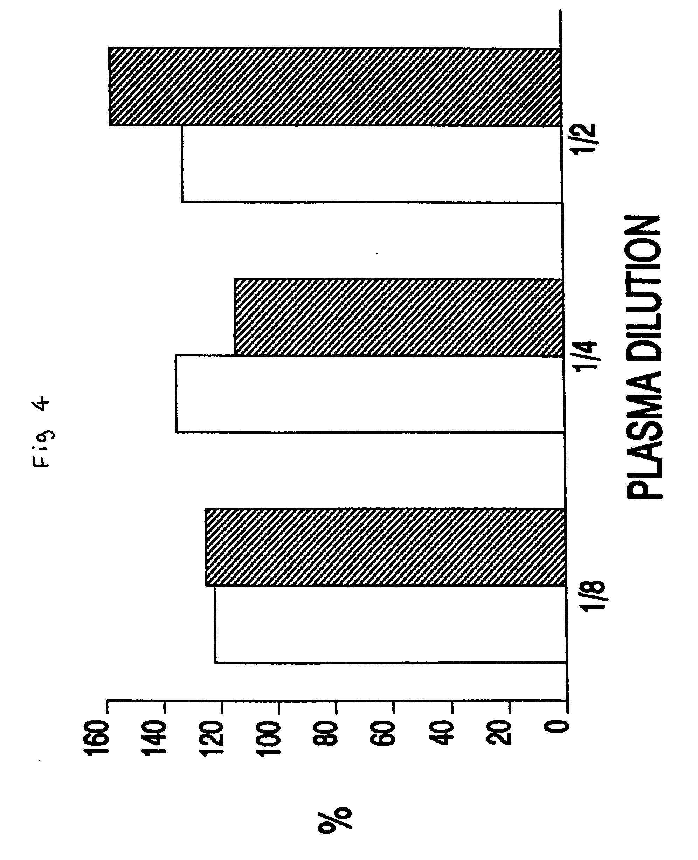

A) Leech hyaluronidase (200 IU/ml) was pre-incubated for one hour with dilutions of normal pooled citrated plasma in 20mM MES, 0.1M NaCl pH5.0 buffer and then added to hyaluronic acid (5mg/ml) for a further one hour at 37°C to determine reducing sugar activity (see Example 1 for method). The results, expressed as percent activity in the absence of plasma after subtraction of blanks are shown in Figure 3 of the accompanying drawings. They show that the activity of the leech hyaluronidase is stimulated by the presence of plasma.
B) Leech hyaluronidase (200 IU/ml) was pre-incubated for one hour at 25°C with and without plasma and heparinised plasma in 20mM MES, 0.1M NaCl, pH5.0 buffer. Plasma was obtained from a patient before and after heparinisation with 30,000 USP/24h resulting in an increase in the clotting time of 40 seconds to 80 seconds. Incubations were continued by addition to hyaluronic acid (see section A, above) and reducing sugars determined as above (Example 1).
   The results indicated no significant inhibition of leech hyaluronidase activity in heparinised plasma compared with non-heparinised plasma from the same patient, as shown in Figure 4 (in which columns A represent runs without heparin and columns B represent runs with heparin).

### Example 5

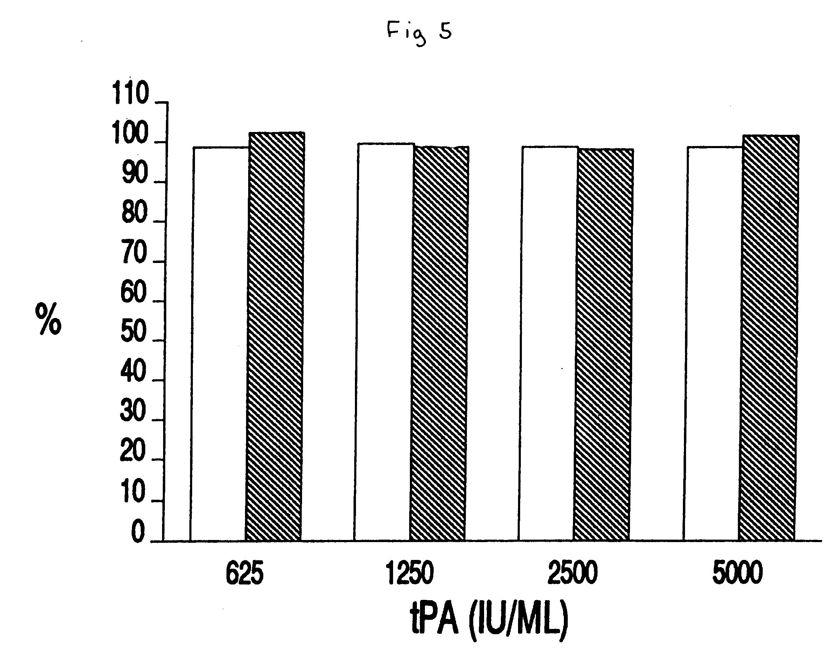

A) Leech hyaluronidase (68IU/ml) was mixed with and without heparin (200USP/ml) and human tissue plasminogen activator (tPA) in a range of concentrations (0.5000 IU/ml) and with hyaluronic acid (5mg/ml) and incubated for 1h at 37°C. Reducing sugars were determined (Example 1) and results expressed as a percentage of controls containing no tPA.
   The results showed no effect of tPA on leech hyaluronidase activity either in the presence or absence of a relatively high dose of heparin, as shown in Figure 5, in which columns A represent runs without heparin and columns B represent runs with heparin.
B) Leech hyaluronidase (75 IU/ml) was mixed with and without hementin (180IU/ml) (where IU is defined as one microgram of fibrinogen incoagulated/min/37°C) and incubated with heparin (1.5 - 1500 USP/ml) for one hour at 25°C prior to addition to hyaluronic acid (2mg/ml) and further incubation for one hour at 37°C. Reducing sugar levels were determined (see Example 1) and the results expressed as a percentage of controls containing no heparin. (Table 3).

**Table 3**

| | Heparin (USP/ml) | % Control |
|---|---|---|
| Without Hementin | 1.5 | 97.5 |
| | 15 | 100 |
| | 150 | 103 |
| | 1500 | 103 |
| With Hementin | 1.5 | 103 |
| | 15 | 93.8 |
| | 150 | 100 |
| | 1500 | 109 |

The results indicate substantially no effect of hementin on leech hyaluronidase activity in the presence or absence of heparin.

## Claims

1. A pharmaceutical formulation comprising heparin in combination with an endo-beta-glucuronidase.

2. A formulation according to claim 1, which further comprises a pharmacologically acceptable diluent carrier or excipient.

3. A formulation according to claim 1 or 2, wherein said endo-beta-glucuronidase is leech-derived.

4. A formulation according to any of claims 1 to 3, which further comprises at least one clot-lytic agent.

5. A formulation according to claim 4, wherein said clot-lytic agent comprises tissue plasminogen activator and/or hementin.

6. A formulation according to any of claims 1 to 5, which is in a form suitable for intravenous administration.

7. An injectable unit dose, which comprises a sealed sterile ampoule containing a formulation according to claim 6.

8. The use of an endo-beta-glucuronidase for the manufacture of a medicament for the therapy of a heparin-treated patient.

9. The use of an endo-beta-glucuronidase according to claim 8 in combination with hementin and/or in combination with a tissue plasminogen activator.

## Patentansprüche

1. Eine pharmazeutische Formulierung, enthaltend Heparin in Kombination mit einer Endo-beta-Glucuronidase.

2. Eine Formulierung nach Anspruch 1, die außerdem einen pharmakologisch akzeptierbaren Verdünner, Träger oder Exzipient enthält.

3. Eine Formulierung nach Anspruch 1 oder 2, wobei die genannte Endo-beta-Glucuronidase von Blutegeln abgeleitet ist.

4. Eine Form nach einem der Ansprüche 1 bis 3, die außerdem zumindest ein Klümpchen-lysierendes Mittel enthält.

5. Eine Formulierung nach Anspruch 4, wobei das Klümpchenlysierende Mittel Gewebe-Plasminogen-Aktivator und/oder Hementin enthält.

6. Eine Formulierung nach einem der Ansprüche 1 bis 5 in einer für die intravenöse Verabreichung geeigneten Form.

7. Eine injizierbare Einheitsdosis, enthaltend eine versiegelte sterile Ampulle, enthaltend eine Formulierung nach Anspruch 6.

8. Die Verwendung einer Endo-beta-Glucuronidase zur Herstellung eines Medikaments für die Therapie eines Heparin-behandelten Patienten.

9. Die Verwendung einer Endo-beta-Glucuronidase nach Anspruch 8 in Kombination mit Hementin und/oder in Kombination mit einem Gewebe-Plasminogen-Aktivator.

## Revendications

1. Composition pharmaceutique comprenant de l'héparine en combinaison avec une endo-bêta-glucuronidase.

2. Composition selon la revendication 1 qui comprend de plus un excipient ou un support diluant pharmaceutiquement acceptable.

3. Composition selon la revendication 1 ou 2, dans laquelle ladite endo-bêta-glucuronidase est issue d'une sangsue.

4. Composition selon l'une quelconque des revendications 1 à 3 qui comprend de plus au moins un agent anti-coagulant.

5. Composition selon la revendication 4 dans laquelle ledit agent anti-coagulant comprend un activateur du plasminogène tissulaire et/ou de l'hémentine.

6. Composition selon l'une quelconque des revendications 1 à 5 caractérisée en ce qu'elle est sous une forme pouvant être administrée par voie intraveineuse.

7. Dose unitaire injectable qui comprend une ampoule scellée stérile contenant une composition selon la revendication 6.

8. Utilisation d'une endo-bêta-glucuronidase pour la fabrication d'un médicament pour la thérapie d'un patient traité à l'héparine.

9. Utilisation d'une endo-bêta-glucuronidase selon la revendication 8 en combinaison avec de l'hémentine et/ou en combinaison avec un activateur du plasminogène tissulaire.
